# EUROPEAN PATENT SPECIFICATION

(11) **EP 4 173 634 B1**
(45) Date of publication and mention of the grant of the patent: **04.03.2026**
(21) Application number: 21758042.2
(22) Date of filing: 28.06.2021
(51) Int. Cl.: A61K 39/00, C12N 1/00, A61K 39/095

(54) **PROCESS FOR OBTAINING ANTIGEN-PRESENTING VESICLES (APV) THAT ENABLES THE COUPLING OF ONE OR MORE ANTIGENS**
PROZESS ZUR HERSTELLUNG ANTIGENPRÄSENTIERENDER VESIKEL (APV), WELCHER DIE BINDUNG EINES ODER MEHRERER ANTIGENE ERMÖGLICHT
PROCÉDÉ DE PRODUCTION DE VÉSICULES PRÉSENTATNT DES ANTIGÈNES PERMETTANT LA FIXATION D'UN OU PLUSIEURS ANTIGÈNES

(30) Priority: 26.06.2020 BR 102020013216
(43) Date of publication of application: 03.05.2023
(73) Proprietor: INSTITUTO BUTANTAN, 05503900 São Paulo - SP (BR)
(72) Inventor: BARBOSA, Mayra Mara Ferrari, 18682-344 Lençóis Paulista - SP (BR); BARAZZONE, Giovana Cappio, 06070-080 Osasco - SP (BR); LEITE, Luciana Cezar De Cerqueira, 05083-030 São Paulo - SP (BR)
(74) Representative: De Clercq & Partners
(86) International application number: PCT/BR2021/050284
(87) International publication number: WO 2021/258180

(56) References cited:
- WO-A1-03/051379
- WO-A1-2011/110634
- US-A1- 2007 166 333
- ANONYMOUS: "Carbodiimide Crosslinker Chemistry | Thermo Fisher Scientific - NL", 1 January 2021 (2021-01-01), XP055853091, Retrieved from the Internet <URL:https://www.thermofisher.com/nl/en/home/life-science/protein-biology/protein-biology-learning-center/protein-biology-resource-library/pierce-protein-methods/carbodiimide-crosslinker-chemistry.html> [retrieved on 20211020]
- H BART VAN DEN BERG VAN SAPAROEA ET AL: "Display of Recombinant Proteins on Bacterial Outer Membrane Vesicles by Using Protein Ligation", APPLIED AND ENVIRONMENTAL MICROBIOLOGY, vol. 84, no. 8, 9 February 2018 (2018-02-09), pages e02567 - 17, XP055616626, DOI: 10.1128/AEM.02567-17
- DANIEL HATLEM ET AL: "Catching a SPY: Using the SpyCatcher-SpyTag and Related Systems for Labeling and Localizing Bacterial Proteins", INTERNATIONAL JOURNAL OF MOLECULAR SCIENCES, vol. 20, no. 9, 30 April 2019 (2019-04-30), pages 2129, XP055618464, DOI: 10.3390/ijms20092129
- LIAO TING-YU ANGELA ET AL: "Improving the Immunogenicity of the Mycobacterium bovis BCG Vaccine by Non-Genetic Bacterial Surface Decoration Using the Avidin-Biotin System", PLOS ONE, vol. 10, no. 12, 30 December 2015 (2015-12-30), pages e0145833, XP055853095, DOI: 10.1371/journal.pone.0145833
- ZHOU YI ET AL: "Fabrication of cell-derived biomimetic drug delivery system", NANOFABRICATION, vol. 5, no. 1, 28 June 2019 (2019-06-28), pages 1 - 18, XP055853100, DOI: 10.1515/nanofab-2019-0001
- JIANG LINGLEI ET AL: "A post-insertion strategy for surface functionalization of bacterial and mammalian cell-derived extracellular vesicles", BIOCHIMICA ET BIOPHYSICA ACTA, vol. 1865, no. 4, 14 October 2020 (2020-10-14), AMSTERDAM, NL, pages 129763, XP055853110, ISSN: 0304-4165, DOI: 10.1016/j.bbagen.2020.129763

## Description

### Application field:

The present disclosure falls within the field of application of preparations for medical purposes, more specifically, in the field of medicinal preparations containing antigens or antibodies, since it refers to a process for obtaining antigen-presenting vesicles (APV) that enables the coupling of one or more antigens for the preparation of immunogenic compositions, such as vaccines, and immunotherapeutic compounds.

### Background and State of the Art:

Outer membrane vesicles (OMV) are structures produced by Gram-negative bacteria. These spherical structures are formed from the outer membrane of Gram-negative bacteria and their periplasm. OMVs associate with soluble and insoluble proteins and perform several biological functions.

These vesicles can function as a potent adjuvant in vaccines, given the presence of lipopolysaccharides (LPS) and proteins on their surface, and also as an important carrier.

By knowing this, the present disclosure proposes a process for obtaining antigen-presenting vesicles (APV) which comprises an immunologically effective amount of at least one OMV, an immunologically effective amount of at least one antigenic protein or peptide, and at least a pair of molecules with complementary affinities (for example, a biotin or a derivative and a protein with affinity to biotin), thus obtaining an antigen-presenting vesicle (APV) that enables the coupling of one or more antigens for the preparation of immunogenic compositions, such as vaccines, and immunotherapeutic compounds.

However, there is a technical problem in the prior art regarding typical conjugation technology, which involves a rigorous treatment of proteins that can result in several difficulties, such as the fact that it increases the risk of denaturation and other modifications of the antigen protein or peptide and the fact that unnecessary modifications / damage to the main structure of the vesicles occur.

In order to solve the existing technical problem, the present disclosure proposes a process that unexpectedly avoids the risk of denaturation and other modifications of the antigen, thus providing a substantial advantage in preserving the antigenicity of the included proteins.

Likewise, the process of the present disclosure prevents unnecessary modifications / damage of the main structure of the vesicles because there is no heavy chemical interjection: biotinylation can be controlled to react with specific functional groups in the vesicle. This control can be done by choosing the conjugation reagent as well as the biotin or derivatives to be used. In one example, EDAC, activator molecule, reacts with the carboxylic groups, activating them and making them available for conjugation with the amine groups of the biotin derivative. As the only component directly conjugated to OMVs is biotin, no other parallel conjugation methodologies are needed, as the antigens will always have affinity to biotin.

The state of the art describes processes for obtaining immunotherapeutic compositions that enable the coupling of one or more antigens.

Brazilian patent application no. BR 11 2013 028887 6, published on August 22, 2017, in the name of CHILDREN'S MEDICAL CENTER CORPORATION, entitled: "IMMUNOGENIC COMPOSITION PRESENTING MULTIPLE ANTIGENS, AND METHODS AND USES OF THE SAME" describes an immunogenic composition comprising an immunologically effective amount of at least one antigenic polysaccharide, an immunologically effective amount of at least one peptide or polypeptide antigen, and at least a pair of complementary affinity molecules, their uses and a kit. Differently, the present disclosure reveals a process for obtaining an antigen-presenting vesicle (APV) that enables the coupling of one or more antigens for the preparation of immunogenic compositions, such as vaccines, and immunotherapeutic compounds through the biotinylation of OMVs and that does not use antigenic polysaccharides as in the document BR 11 2013 028887 6. It is important to emphasize that this document does not disclose the use of OMVs, as proposed by the present disclosure.

Van den Berg van Saparoea et al. (Applied and Environmental Microbiology, 2018) and Hatlem et al. (International Journal of Molecular Sciences, 2019) disclose OMVs from Salmonella which carry the *E. coli* virulence factor hemoglobin protease (Hbp) fused to the affinity tags SpyTag, SpyCatcher, SnoopTag or SnoopCatcher. In these publications desired antigens are fused to the corresponding complement.

Angela Liao et al. (Plos One, 2015) report an improvement of the *M. bovis* BCG vaccine by non-genetic bacterial surface decoration using an avidin-biotin system, which is presented by the authors as an alternative to DNA-based gene expression for improving the current BCG vaccine.

Zhou *et al.* provide an overview of different processing methods for engineering cell-derived biomimetic drug delivery systems.

US 2007/166333 A1 describes a method for the insertion of protein antigens in outer membrane vesicles of Gram-negative bacteria without disruption of the vesicle structure. The described method is able to provide in membrane vesicle suitable for application in the pharmaceutical industry as vaccines.

WO 03/051379 A1 discloses a composition of a mixture of different vesicles such as OMVs. In addition, the document discloses a composition containing in a single vesicle a combination of antigens and/or other vesicle components derived from separate vesicles.

WO 2011/110634 A1 provides Neisserial immunogenic compositions and vaccines based on the use of chimeric factor H binding protein (fHbp) proteins to overcome to avoid the problem encountered by earlier compositions wherein the immune responses specificity is limited to the meningococcal strain from which the fHbp was derived.

Finally, Jiang et al. (Biochimica et Biophysica Acta, 2020) discloses extracellular vesicles such as OMVs into which biotin is integrated without describing complements fused to avidin in order to produce antigen presenting vesicles.

Therefore, no prior art document describes or suggests a process for obtaining of an antigen-presenting vesicle (APV) composed of an outer membrane vesicle of gram-negative bacteria (OMV) that enables the coupling of one or more antigens for the preparation of immunogenic compositions, such as vaccines, and immunotherapeutic compounds.

### Summary of the disclosure:

The invention is defined in the appending claims.

The present disclosure will provide significant advantages in the field of medicinal preparations containing antigens or antibodies. The scope of the invention is limited to the claims appended hereto.

In a first aspect, the present disclosure refers to a process for obtaining antigen-presenting vesicles (APV) that enables the coupling of one or more antigens, wherein such APV comprises (i) an outer membrane vesicle of gram-negative bacteria (OMV); (ii) at least one antigenic protein or peptide; and (iii) at least a pair of molecules with complementary affinities comprising a first affinity molecule that associates with the vesicle, and a complementary affinity molecule that associates with the protein or peptide.

Thus, the process for obtaining the APV of the present disclosure is essential to achieve a form of presentation that consists of a vesicle that makes it possible to attach one or more proteins, or a plurality of different protein or peptide antigens.

In that regard, such process comprises the following steps:
a) Conjugating the first affinity molecule (biotin or a derivative) to the vesicle (OMV), optionally with the aid of an activator molecule;
b) Genetically fusioning the complementary affinity molecule (avidin or a derivative such as rizavidin) to protein antigens or peptides; and
c) Coupling the fusion protein obtained in step "b" with the product obtained in step "a".

### Brief description of the figures:

The structure and operation of the present disclosure, together with additional advantages thereof, can be better understood by referring to the attached images and the following description.
Figure 1 represents an illustrative image of a representative schematic of the assembly of the APV complex with the recombinant antigen (rAg).
Figures 2A - B graphically represent the purification of the APV-rAg Complex, in this case rAg is the *Schistosoma mansoni* protein, rSmTSP-2, wherein (A) is the analysis of the fractions of the gel filtration chromatography of the APV-rSmTSP-2 complex in a spectrophotometer, and (B) is the *Western blot* of the fractions that make up Peak A, revealed with anti-rSmTSP-2 antibody.
Figures 3A - D represent the biophysical characterization of the APV-rSmTSP-2 complex, wherein (A) is a transmission electron microscopy (TEM) image of OMVs isolated from *Neisseria lactamica;* (B) is a TEM image of detoxified OMVs; (C) is a TEM image of the APV-rSmTSP-2 complex, wherein the size bars in the right corner are indicated on each image; and (D) are the size distribution curves of OMVs isolated from *N. lactamica,* detoxified OMVs and APV-rSmTSP-2 complex.
Figure 4 graphically represents cytokine production in supernatant of splenocytes stimulated after 2 or 3 doses of APV-rSmTSP-2.
Figure 5 graphically represents the production of cytokines by T-CD4+ and T-CD8+ cells from mice immunized with APV-rTSP-2.
Figures 6A - B graphically represent the IgG humoral response against the rSmTSP-2 protein in mice immunized with rSmTSP-2 or APV-rSmTSP-2, wherein (A) represents the dosage of anti-rSmTSP-2 IgG antibodies in immunized mice, and (B) are the IgG1 and IgG2c isotypes in the groups immunized with rSmTSP-2 or APV-rSmTSP-2.

### Detailed description of the disclosure:

Even though the present disclosure may be susceptible to different examples, the drawings and the following detailed discussion show a preferred example with the understanding that the present example is to be considered an exemplification of the principles of the disclosure and is not intended to limit the present disclosure to what has been illustrated and described in this report.

The present disclosure refers to a process for obtaining antigen-presenting vesicles (APV) that enables the coupling of one or more antigens, wherein such APV comprises:
- an outer membrane vesicle of gram-negative bacteria (OMV), in a preferred example, it is used OMV derived from *N. lactamica;*
- at least one antigenic protein or peptide; e
- at least a pair of molecules with complementary affinities comprising:
   (i) a first affinity molecule that associates with the vesicle (such as a biotin or biotin derivative); and
   (ii) a complementary affinity molecule that associates with the protein or peptide (such as avidin or a derivative) .

Thus, complementary affinity molecules serve as an indirect *link* between the vesicle and the antigenic protein or peptide.

The process for obtaining the APV of the present disclosure is essential to achieve a form of presentation that consists of a vesicle that makes it possible to attach one or more proteins, or a plurality of different protein or peptide antigens.

In that regard, such process comprises the following steps:
a) Conjugating the first affinity molecule (biotin or derivative) to the vesicle (OMV), optionally with the aid of an activator molecule;
b) Obtaining the antigen protein(s) or peptide(s) fusion with the complementary affinity molecule; and
c) Coupling the fusion protein obtained in step "b" with the product obtained in step "a".

In step "a", the conjugation reaction of the OMVs with the first affinity molecule is carried out in a suitable solution with the addition of 3 % sucrose, in which OMVs are added in the proportion of 1:1 to 1:10 (mass / mass) in relation to the first affinity molecule, and optionally 0,05 - 0,2 M of activator molecule.

The suitable solution used depends on the type of conjugation used. Suitable solutions include but are not limited to phosphate-buffered saline solution without Ca²⁺ / Mg²⁺ (PBS) or normal saline (150 mM NaCl in water), all with the addition of 3 % sucrose to stabilize OMV membranes. Still, several buffers can be used in the aforementioned appropriate solution, however, these buffers must contain the ideal pH range and have no interfering agent for conjugation, such as EDTA or glycine.

Such mixture it is then kept at a temperature of 4 to 25 °C for 4 to 18 hours. The mixture then proceeds to dialysis against the appropriate solution previously used to eliminate the excess of the first unbound affinity molecule. The most commonly used solutions for such elimination include, but are not limited to, phosphate-buffered saline solution without Ca²⁺ / Mg²⁺ (PBS), normal saline solution (150 mM NaCl in water) and Tris buffer.

In step "a", such OMVs come from bacteria selected from the group consisting of *N. meningitidis* serogroup B or *N. lactamica.* Preferably, the OMVs come from *N. lactamica* N.285/03.

Still in step "a", such first affinity molecule is selected from the group consisting of biotin, a biotin derivative, or a biotin mimic, for example, but not limited to amine-PEG3-biotin ((+)-biotinyl-3-6,9-trioxaundecandiamine) or a derivative or functional fragment thereof, preferably amine-PEG3-biotin ((+)-biotinyl-3-6,9-trioxaundecandiamine), more preferably biotin.

Still in step "a", such activator molecule is selected from the group consisting of representative coupling agents that include organic compounds, such as thioesters, carbodiimides, succinimide esters, diisocyanatos, glutaraldehydes, diazo benzenes and hexamethylene diamines.

As explained above, it is worth noting that the use of an activator molecule is optional, wherein its use depends on the first affinity molecule used. For example, if a modified biotin is used as first affinity molecule, an activator molecule, such as EDAC (1-Ethyl-3-[3-dimethylaminopropyl] carbodiimide) hydrochloride is necessary. However, if another biotin is used, such as an NHS-Biotin, the reaction would take place with OMV's own NH₂-groups, not requiring the use of an activator molecule.

In that regard, alternatively, in other examples, the first affinity-binding molecule may contain functional groups that bind directly to OMV, without the aid of activating molecules.

In another example, said activator molecule is used to covalently bind the first affinity binding molecule to the vesicle (OMV).

Thus, the first affinity molecule can be attached to the carboxyl, hydroxyl, amino, phenoxy, hemiacetal or mercapto functional groups of the OMV, depending on the conjugation chemistry selected.

In step "b" a fusion protein comprising the antigen protein or peptide fused to the complementary affinity molecule is obtained.

To this end, the complementary affinity molecule is genetically fused to antigen proteins or peptides through the recombinant construction joining the gene of complementary affinity molecule to the gene of the antigen or peptide of interest. Molecular biology techniques such as using conventional polymerase chain reaction (PCR), or gene synthesis can be used to construct a chimeric sequence that encodes a fusion protein.

Such antigen protein or peptide are selected from the group consisting of any antigen that triggers an immune response in an organism, including, but not limited to, immunogenic peptides or proteins, toxins, toxoids, their subunits, or combinations thereof (for example, cholera toxin, tetanus toxoid).

Additionally, the antigen, which is fused to complementary affinity molecule, is selected from the group consisting of any antigen associated with an infectious disease or cancer or immune disease. In some examples, the antigen may be a component expressed by any of a variety of infectious agents, including viruses, bacteria, fungi, or parasites.

In some examples, the antigen is derived (for example, obtained) from a pathogenic organism. In some examples, the antigen is a cancer or tumor antigen, for example an antigen derived from a tumor or cancer cell.

In some examples, an antigen derived from a pathogenic organism is an antigen associated with an infectious disease; can be derived from any of a variety of infectious agents, including viruses, bacteria, fungi, or parasites.

A target antigen for use in the process described herein may be expressed by recombinant means and may optionally include an affinity tag or epitope to facilitate purification, which methods are well known in the art. Chemical synthesis of an oligopeptide, free or conjugated to carrier proteins, can be used to obtain the antigen of the disclosure. Oligopeptides are considered a type of polypeptide.

The antigen can be expressed as a merger with a complementary affinity molecule, for example, but not limited to rizavidin or a functional derivative or fragment thereof. Alternatively, it is also possible to prepare the antigen target and then conjugate it with a complementary affinity molecule, for example, but not limited to rizavidin or a functional derivative or fragment thereof.

An antigen can be obtained by recombinant means or chemical polypeptide synthesis, as well as antigen obtained from natural sources or extracts, it can be purified by means of the physical and chemical characteristics of the antigen from techniques known in the state of the art, such as, for example, by fractionation or chromatography.

In some examples, an antigen can be solubilized in water, solutions, or a buffer. Suitable buffers include but are not limited to phosphate-buffered saline solution without Ca²⁺ / Mg²⁺ (PBS), normal saline solution (NaCl 150 mM in water) and Tris buffer. The antigen not soluble in neutral buffer can be solubilized in 10 mM acetic acid and then diluted to the desired volume with a neutral buffer such as PBS. In the case of the antigen soluble only at acidic pH, acetate-PBS at acidic pH can be used as a diluent after solubilization in dilute acetic acid. Glycerol may be a suitable non-aqueous solvent for use in the process described herein.

Recombinant antigens can be synthesized and purified by protein purification methods using bacterial expression systems, yeast expression systems, baculovirus / insect cell expression system, mammalian cell expression systems, or plant systems or transgenic animals, as they are known by the person skilled in the art.

Fusion proteins can be synthesized and purified by proteinaceous and molecular methods that are well known to those skilled in the art. Molecular biology methods and recombinant heterologous protein expression systems are used. For example, a recombinant fusion protein can be expressed in host cells such as bacteria, mammals, insects, yeasts, or plants, or in transgenic plants or animal hosts.

In one example, in step "b", an isolated polynucleotide encodes a fusion protein. Conventional polymerase chain reaction (PCR) cloning techniques can be used to construct a chimeric sequence that contains the gene or gene fragment encoding the biotin binding protein, a binding sequence *("linker")* and the gene encoding the antigen, which together encodes a fusion protein, which consists of the joining of two proteins, as described herein. It is important to emphasize that the fusion protein is the genetic construct that joins two proteins that naturally have individual genetic codes that code for specific proteins that will be produced separately, but that when fused, they have a genetic code that codes for the production of these two proteins together and linked.

A coding sequence can be cloned into a general-purpose cloning vector such as, but not limited to pUC19, pBR322, pBLUESCRIPT^{®} (Stratagene, Inc.) or pCR TOPO^{®} (Invitrogen) vectors. Unfused proteins contain only one gene in their construction, that is, a single genetic code, a unique sequence that codes for a single protein.

In some examples, in step "b", wherein the antigen is fused with a complementary affinity molecule, the signal sequence may be located at the N-terminal portion of the complementary affinity molecule.

For example, if an antigen is fused to an avidin-like molecule, the signal sequence can be located in the N-terminal portion of the complementary affinity molecule. In some examples, the signal sequence is cleaved from the complementary affinity molecule before the association of the complementary affinity molecule to the first affinity molecule.

Still in step "b", the complementary affinity molecule is selected from the group consisting of avidin, rizavidin, streptavidin or variants, derivatives, or functional portions thereof.

In some examples, the complementary affinity molecule is an avidin-related polypeptide. In specific examples, the complementary affinity molecule is rizavidin, such as recombinant rizavidin. In particular, recombinant rizavidin is modified so that it can be expressed in *E. coli* with high yield. The typical yield is > 30 mg per liter of *E. coli* culture. Rizavidin has a lower sequence homology than egg avidin (22,4 % of sequence identity and 35.0 % similarity) compared to other avidin-like proteins. The use of modified rizavidin reduces the risk of APV inducing an egg-related allergic reaction in an individual. Furthermore, the antibody against recombinant modified rizavidin does not show apparent cross-reactivity to egg avidin (and vice versa).

In step "c" the coupling of the biotinylated OMVs obtained is performed in step "a" with the fusion proteins obtained in step previous.

To carry out the coupling of biotinylated OMVs with fusion proteins, the conjugated product OMV-first affinity molecule is mixed with the obtained fusion proteins in step "b" in the proportion of 1:1 (mass / mass). This incubation takes place at a temperature ranging from 4 to 25 °C for 4 to 18 hours. After incubation, the mixture is centrifuged at 3000 to 14000 rpm for 3 to 30 minutes to remove insoluble aggregates. The supernatant is then purified.

Ultrafiltration can be used for purification of the APV product obtained in step "c" through membranes of different pore sizes (for example AMICON^{®} or MILLIPORE^{®} membranes). The mixture containing the APVs is ultrafiltered through a membrane with a pore size smaller than the size of the APV. The ultrafiltration retentate will contain the APVs, which can then be subjected to chromatography.

The APV product obtained in step "c" can also be separated from other proteins based on their size, surface charge, hydrophobicity, and affinity for ligands. In some examples, a combination of purification steps comprising, for example: (a) ion exchange chromatography, (b) affinity chromatography, (c) hydrophobic interaction chromatography and (d) size exclusion chromatography can be used to purify the obtained APV.

All of these methods are well known in the prior art. It will be evident to a person skilled in the art that chromatographic techniques can be performed at any scale and using equipment from many different manufacturers.

Therefore, surprisingly, the process herein described allows the obtainment of an antigen-presenting vesicle (APV) composed of an outer membrane vesicle of gram-negative bacteria (OMV) that enables the coupling of one or more antigens for the preparation of immunogenic compositions, such as vaccines, and immunotherapeutic compounds.

### Definitions

It should be understood that this disclosure is not limited to specific methodology, protocols, and reagents, etc. used in the process described herein and as such may vary. The terminology used in this document is intended to describe only particular examples and is not intended to limit the scope of the present disclosure.

All technical and scientific terms used herein have the same meaning as is commonly understood by a person skilled in the art to which this disclosure belongs.

The term "antigen-presenting vesicle (APV)" used herein is defined as a platform that allows the delivery of the antigen for efficient activation of the immune system.

The term "immunogenic composition" used herein is defined as a composition capable of eliciting an immune response, such as an antibody or cellular immune response, when administered to an individual. The immunogenic compositions of the present disclosure may or may not be immunoprotective or therapeutic. When the immunogenic compositions of the present disclosure prevent, ameliorate, alleviate, or eliminate disease in the subject, then the immunogenic composition may optionally be referred to as a vaccine. As used herein, however, the term immunogenic compositions is not intended to be limited to vaccines.

As used herein, the term "antigen" refers to any substance that elicits an immune response directed against the substance. In some examples, an antigen is a peptide or polypeptide, and in other examples, it can be any chemical substance or fraction, for example, a carbohydrate, that elicits an immune response directed against the substance.

The term "associated" or "coupled", as used herein, refers to the linking of two or more molecules by non-covalent bonds. In some examples, where the bond of two or more molecules occurs by a non-covalent bond, the two or more molecules can form a complex.

The term "complex", as used herein, refers to a collection of two or more molecules, spatially connected by means other than a covalent interaction; for example, they can be connected by electrostatic interactions, hydrogen bonds, or by hydrophobic interactions (i.e., *van der Waals* forces).

The term "conjugation reaction" or "conjugation", as used herein, refers to a covalent bond formed between a polymer chain and a second molecule. The term "activator molecule" refers to a component that is an intermediate molecule to favor the covalent bonding of a polymer or OMV with another molecule, for example, the first affinity molecule (biotin or a derivative).

As used herein, the term "fused" or "fused" means that at least one protein or peptide is physically associated with a second protein or peptide. In some examples, the fusion is typically a covalent bond. Covalent bonding can encompass binding as a fusion protein or chemically coupled bond, for example, via a disulfide bond formed between two cysteine residues.

As used herein, the term "fusion protein" means a protein created by joining two or more polypeptide sequences. Fusion proteins encompassed by this disclosure include translation products of a chimeric gene construct that joins the DNA sequences encoding one or more antigens, or fragments or mutants thereof, with the DNA sequence encoding a second polypeptide to form a single open reading frame. In other words, a "fusion protein" is a recombinant protein of two or more proteins that are joined by a peptide bond or through several peptides. In a preferred example, the second protein to which the antigens are fused is a complementary affinity molecule which is able of interacting with a first affinity molecule of the complementary affinity pair.

The terms "polypeptide" and "protein" can be used interchangeably to refer to a polymer of amino acid residues linked by peptide bonds and, for the purposes of the claimed disclosure, have a typical minimum length of at least 25 amino acids. The term "polypeptide" and "protein" may encompass a multimeric protein, for example, a protein containing more than one domain or subunit. The term "peptide", as used herein, refers to an amino acid sequence linked by peptide bonds containing less than 25 amino acids, for example, between about 4 amino acids and 25 amino acids in length. Proteins and peptides can be composed of amino acids arranged linearly linked by peptide bonds, whether produced biologically, recombinantly or synthetically, and compounds of naturally occurring or non-naturally occurring amino acids are included in this definition. Full-length proteins and their fragments longer than 25 amino acids are encompassed by the definition of protein. The terms also include polypeptides that have post-translational (for example, signal peptide cleavage) and post-translational polypeptide modifications, such as, for example, disulfide bond formation, glycosylation, acetylation, phosphorylation, lipidation, proteolytic cleavage (for example, cleavage by metalloproteases) and the like. Also, as used herein, a "polypeptide" refers to a protein that includes modifications, such as deletions, additions, and substitutions (generally of a conservative nature, as would be known to a person skilled in the art) in the native sequence, as long as the protein maintains the desired activity. These modifications may be deliberate, such as through site-directed mutagenesis, or they may be accidental.

The term "functional fragment", as used in the context of a "functional portion of an antigen", refers to a portion of the antigen or polypeptide of the antigen that mediates the same effect as the complete portion of the antigen, for example, causes an immune response in an individual or mediates an association with another molecule, for example, comprises at least one epitope.

The term "cytokine", as used herein, refers to a molecule released from an immune cell in response to stimulation with an antigen. Examples of such cytokines include, but are not limited to: GM-CSF; IL-1 α; IL-1β; IL-2; IL-3; IL-4; IL-5; IL-6; IL-7; IL-8; IL-10; IL-12; IL-17A, IL-17F or other family members IL-17, IL-22, IL-23, IFN-α; IFN- α; IFN-y; MIP-1a; MIP-1β; TGF-β; TNF-α or TNF-ββ. The term "cytokine" does not include antibodies.

As used herein, the term "pathogen" refers to an organism or molecule that causes a disease or disorder in an individual. For example, pathogens include, but are not limited to, viruses, fungi, bacteria, parasites, and other infectious organisms or molecules, as well as taxonomically related macroscopic organisms in the algae, fungi, yeasts, protozoa categories, or the like.

The term "native" refers to the naturally occurring normal polynucleotide sequence that encodes a protein, or a portion thereof, or protein sequence, or portion thereof, respectively, as it normally exists *in vivo.*

The term "mutant" refers to an organism or cell with any change in its genetic material, in particular a change (i.e., deletion, substitution, addition, or alteration) relating to a wild-type polynucleotide sequence or any relative change to a wild type of protein. The term "variant" can be used interchangeably with "mutant". Although a change in genetic material is often assumed to result in a change in protein function, the terms "mutant" and "variant" refer to a change in the sequence of a wild-type protein, regardless of whether that change alters the function of the protein (e.g., increases, decreases, confers a new function) or if that change does not affect the function of the protein (e.g. the mutation or variation is silent).

As used herein, the term "heterologous" refers to sequences of nucleic acids, proteins, or polypeptides and means that these molecules are not naturally occurring in that cell. For example, the nucleic acid sequence encoding a fusion antigen protein described herein that is inserted into a cell, e.g., in the context of a protein expression vector, is a heterologous nucleic acid sequence.

The term "recombinant", when used to describe a nucleic acid molecule, means a polynucleotide of genomic, cDNA, viral, semi-synthetic and/or synthetic origin, which, by virtue of its origin or manipulation, is not associated with all or a portion of the polynucleotide sequences with which it is associated in nature. The term recombinant as used in relation to a recombinant peptide, polypeptide, protein, or fusion protein, means a polypeptide produced by expression of a recombinant polynucleotide. The term recombinant, as used in relation to a host cell, means a host cell into which a recombinant polynucleotide has been introduced. Recombinant is also used herein to refer, with reference to the material (e.g., a cell, a nucleic acid, a protein, or a vector) that the material has been modified by the introduction of a heterologous material.

The term "host cell" refers to any cell that can receive the fusion protein expression vector and express the recombinant protein. The host cell can be bacterial, mammalian, insect, yeast, or plant cells, or in transgenic plants or animal hosts.

### Preferred Examples of the Disclosure:

### - Production of outer membrane vesicles (OMVs)

The *N. lactamica* strain used was No.285/03. Samples kept in cryotubes at -80 °C were thawed at 36 °C and the bacterial suspension was used to inoculate tubes containing Müller-Hinton agar. These tubes were kept inclined at 36 °C, in a CO₂ atmosphere for 24 h. After this first cell activation culture, the cell mat formed on the agar was resuspended in 3 mL of MC2LAA-YE medium (5.8 g / L of sodium chloride; 0.40 g / L of ammonium chloride; 0.186 g / L of potassium chloride; 0.037 g / L of calcium chloride dihydrate; 0.647 g / L of sodium citrate; 0.616 g / L of magnesium sulfate heptahydrate; 0.001 g / L of hydrated manganese sulfate; 2.36 g / L of L-glutamic acid; 0.21 g / L of L-arginine.HCl; 0.302 g / L of glycine; 0.042 g / L of L-serine; 0.022 g / L of L-cysteine.HCl.H₂O; 5 ml / L of glycerol; 15.02 g / L of sodium lactate; 1.062 g / L of dibasic sodium phosphate; 0.171 g / L of monobasic potassium phosphate; 2 g / L of ultrafiltered yeast extract).

These 3 mL were expanded to 100 mL of the same medium, placed in a 300 mL Tunair Flask full baffled, and placed on a 200 - 250 rpm rotary shaker at 36 °C for 12 h. This culture was used to inoculate 1 L of MC2LAA-YE medium in a 1 L Tunair flask, which was incubated on a 200 - 250 rpm rotary shaker at 36 °C. The inoculum volume was calculated so that the initial OD₅₄₀ₙₘ of the experiment was approximately 0.15. The OD₅₄₀ₙₘ was followed during the experiment to identify the end of the stationary phase and then the closure of the culture. The culture was centrifuged at 10,000 rpm, at 10 °C, for 45 min and the supernatant was recovered and supplemented with sodium azide at a final concentration of 0.02 % to avoid contamination.

The supernatant was filtered on a PES membrane with a pore size of 0.45 µM and ultracentrifuged at 30,000 rpm, 10 °C, for 3 h. The precipitate was resuspended in 50 ml PBS + 3% sucrose. These 50 mL were then mixed with 50 mL of 100 mM Tris, 2 mM EDTA, 1 % SDC (sodium deoxycholate), pH 8.5, and this mixture was kept at room temperature for 15 minutes so that the OMVs could be detoxified and followed by tangential ultrafiltration performed with a 100 kDa cut-off membrane in the LabScale equipment (Labscale TFF System - Merck Millipore). In the same equipment, 6 - 10 washes with PBS + 3 % sucrose were performed to eliminate SDC. Finally, the concentration of vesicles was determined by the Bradford colorimetric method.

An aliquot of OMVs was fixed in sodium cacodylate and glutaraldehyde buffer for electron microscopy and evaluation of their integrity.

### Step a - Conjugation of outer membrane vesicles (OMVs) with biotin

Conjugation of OMVs with biotin was performed in PBS buffer, 150 mM NaCl, 3 % sucrose, to which 5 mg of OMV, 10 mg of biotin ((+)-biotinyl-3-6,9-trioxaundecandiamine) and 0,1 M of EDAC (N-(3-Dimethylaminopropyl)-N'-Ethyl carbodiimide hydrochloride - Sigma-Aldrich). This mixture was kept at 4 °C for 18 h. The mixture then went on to dialysis against PBS + 3 % sucrose, to eliminate the excess of unbound biotin. The concentration of OMV-biotin was determined by the Bradford colorimetric method.

### Step b - Construction of recombinant rizavidin-antigen fusion proteins

To construct a fusion protein between biotin-binding protein (for example, avidin or rizavidin, Rv) and the antigen (for example, any antigenic protein or peptide or the *Schistosoma mansoni* protein, SmTSP-2), was constructed from a fusion gene containing the rizavidin gene and the antigen gene of interest (SmTSP-2) separated by a DNA sequence encoding a flexible linker region, inserted directly into the 3' end of the protein gene binding to biotin (rizavidin), to help stabilize the fusion protein. The genes encoding the candidate antigens (full length or fragment) were amplified from the genomic DNA of the pathogens by routine PCR procedures, or synthesized, inserted after the binding region and the fusion gene inserted into the expression vector in *E. coli.*

For protein expression, plasmids containing target constructs were transformed into *E. coli,* strain BL21 (DE3), using the standard heat shock procedure. A single colony was picked from the plate (or a glycerol stock was used later) and inoculated into 30 ml of *Terrific broth* (TB) medium containing Ampicillin (Amp+) for a night culture at 37 °C. On day 2, 5 ml of the initial culture was inoculated into 50 ml of TB / Amp+ medium and grown for 3 - 4 h at 37 °C. These 50 mL of the new culture were used as pre-inoculum and, therefore, were inoculated in 1 liter of TB / Amp+ medium, incubated under shaking at 37 °C until reaching OD₆₀₀ = 3. After cooling the medium to 23 °C, a final concentration of 0.6 mM of IPTG was added to the cultures for an overnight induction.

After cultivation, the bacteria were centrifuged (4000 rpm, 10 min at 4 °C) and resuspended in lysis buffer (150 mM of Tris-HCl pH 7.5; 500 mM of NaCl; 1 mM of PMSF) to perform the mechanical lysis in a high-pressure homogenizer (Panda PLUS 1000 GEA Lab Homogenizer). After lysis, the suspension was centrifuged for 50 min at 12,000 rpm to separate the soluble and insoluble fraction. Soluble proteins went straight to purification. Insoluble proteins were solubilized with 150 mM of Tris-HCl buffer pH 7.5; 500 mM of NaCl; 1 mM of PMSF; 8M of urea. Proteins were purified on a 5 mL HisTrap HP Sepharose column (GE Healthcare), following the manufacturer's instructions, using the Äkta Prime Plus equipment (Amersham Pharmacia).

Recombinant proteins were detoxified to remove lipopolysaccharides (LPS) from *E. coli* through the Triton X-114 wash method. Each 1 ml of protein was mixed with 10 µl of Triton X-114, shaken vigorously and incubated at 37 °C for 15 min for phase separation. After incubation, the samples were centrifuged at 12,000 rpm for 30 seconds and the upper phase was recovered. This wash was repeated three times to ensure the elimination of the LPS. After this step, the protein was quantified by the Bradford colorimetric method.

### Step c - Coupling of OMV-biotin with the Antigens and purification

To perform the coupling of biotinylated OMVs with fusion proteins, the OMV-biotin were mixed with rRvAg in the proportion of 1:1 (mass / mass). This incubation took place at 4 °C for 18 h. After incubation, the mixture was centrifuged at 13,200 rpm for 3 min to remove insoluble aggregates. The supernatant was applied to gel filtration chromatography, using the sepharose-200 column, with PBS, Tris buffer or saline solution as the running solution. Peak fractions containing large molecular weight complex were collected and concentrated. The protein content and the proportion of different antigens in the APV-rAg complex were tested by SDS-PAGE with Coomassie blue staining, and the protein / OMV ratio was determined by *Western blot* using a standard curve with the antigen and specific antibodies.

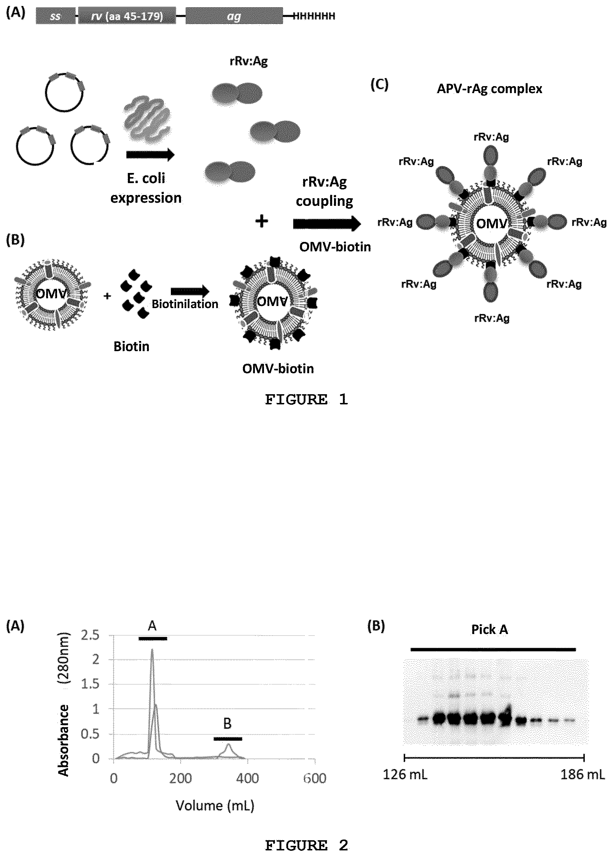

Figure 1 shows the diagram representing the assembly of the APV-rAg complex, wherein (A) is a representation of the expression cassette containing the antigen genes (ag) fused to the rizavidin gene (rv). A secretion signal sequence (ss) is present in the N-terminal region of rv, and after the of the antigen gene has a histidine tail and a representation of the expression in E. *coli* of the Rv-fused proteins; (B) refers to the biotinylation of OMVs; and (C) is a schematic representation of the production of APV-rAg multimolecular complexes.

Figures 2A - B graphically represent the purification of the APV-rSmTSP-2 complex, wherein Figure 2A refers to the analysis of the fractions of the gel filtration chromatography of the APV-rSmTSP-2 complex in a spectrophotometer, 280nm absorbance reading. Still in Figure 2A, peak A refers to the purified complex and peak B refers to protein not bound to OMVs, wherein the calibrator peaks are in blue and the absorbance of the sample fractions during purification is in orange.

Figure 2B refers to the *Western blot* of the fractions that make up Peak A, revealed with anti-rSmTSP-2 antibody.

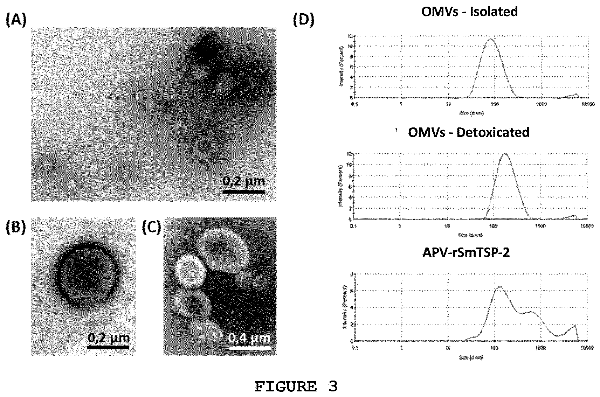

Figures 3A - D illustrate the biophysical characterization of the APV-rSmTSP-2 complex, wherein (A) is a transmission electron microscopy (TEM) image of OMVs isolated from *N. lactamica;* (B) is a TEM image of the detoxified OMVs; (C) is a TEM image of the APV-rSmTSP-2 complex, wherein the size bars in the right corner are indicated on each image; and (D) are the size distribution curves of isolated *N. lactamica* OMVs, detoxified OMVs and APV-rSmTSP-2 complex.

### Tests performed:

### - Immunization, analysis of antibody and cytokine production

C57Bl/6 female mice, obtained from the central vivarium of the Butantan Institute, were kept in the animal testing facility of the Vaccine Development Laboratory according to the rules of the Ethics Committee on the Use of Animals of the Butantan Institute (CEUA / IB) under the CEUA protocol 3314160715.

The vaccines were formulated with sterile saline solution, with the compounds listed below in Table 1. The adjuvant used in this formulation was aluminum hydroxide (Alum), in the proportion (1:10) mass / mass.

**Table 1 - Formulation of groups vaccinated with APV-rSmTSP-2 and controls**

| **Groups** | **Concentrations** |
|---|---|
| Control (Alum) | [90 µg Alum + saline] |
| rSmTSP-2 | [5 µg rSmTSP-2 + 45 µg Alum + saline] |
| OMV | [10 µg OMV + 90 µg Alum + saline] |
| OMV + rSmTSP-2 | [2 µg rSmTSP-2 + 8 µg OMV + 90 µg Alum + saline] |
| APV-rSmTSP-2 | [10 µg APV-rSmTSP-2 + 90 µg Alum + saline] |

The immunization assays followed the scheme of 3 doses applied with an interval of 15 days between them. Serum from these animals was collected before each immunization and also 15 days after the last dose. Thus, it was possible to assess the production of antibodies between each dose. Animals' serums of the same group were analyzed individually by ELISA to verify the total IgG titer and the concentration of IgG1 and IgG2c, specific for the rSmTSP-2 protein.

Splenocytes from immunized mice were isolated and stimulated *"in vitro"* with the recombinant protein rSmTSP-2 or the respective controls. Supernatants were collected and the production of inflammatory cytokines assessed by *Cytometric Bead Array* (CBA - BD biosciences inflammatory kit - US), both following the manufacturer's recommendations.

Table 2 presents the Zetasizer characterization of the OMVs and the APV-rSmTSP-2 complex obtained.

**Table 2 - Characterization of OMVs and APV-rSmTSP-2 complex by ZetaSizer.**

| **Sample** | **Size (nm)** | **Dispersion index (PDI)** | **Zeta potential (mV) ± DP** | **LAL (EU / mL)** |
|---|---|---|---|---|
| **OMV** | 80, 1 | 0,210 | 10,10 ± 0,19 | 12.500.000 > [ ] > 1.250.000 |
| **OMV-detoxified** | 178,5 | 0,203 | -8,09 ± 0,23 | 125.000 > [ ] > 12.500 |
| **APV-rSmTSP-2** | 196, 3 | 0,497 | -28,03 ± 6,07 | 125.000 > [ ] > 12.500 |

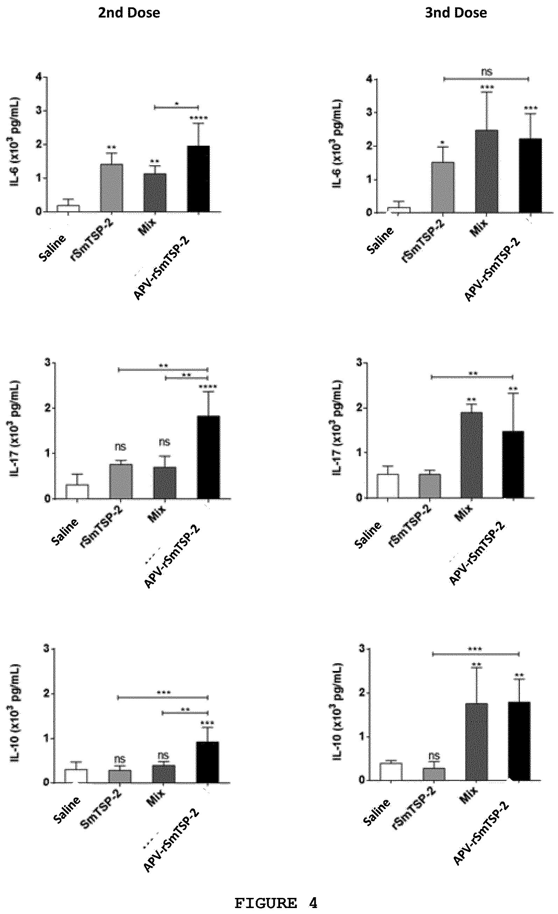

Figure 4 graphically depicts cytokine production in the supernatant of stimulated splenocytes after 2 or 3 doses of APV-rSmTSP-2. Splenocytes isolated from the spleen of mice immunized with APV-rSmTSP-2 were stimulated for 24 h with 10 µg of the rSmTSP-2 protein. Cytokine production was analyzed by CBA in collected supernatants. Statistical analyzes were performed by "One-way ANOVA", wherein P-value *, 0,01; **, 0,001; ***, 0,0001 are values in relation to the saline group, or between the different groups; and ns means no statistical difference.

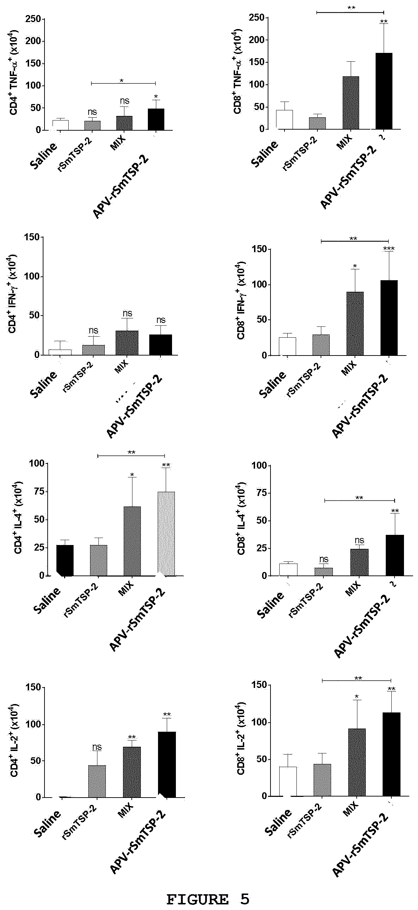

Figure 5 graphically depicts cytokine production by T-CD4+ and T-CD8+ cells from mice immunized with APV-rTSP-2. Splenocytes isolated from the spleen of mice immunized after 3 doses of APV-rSmTSP-2 were stimulated for 6 h with 10 µg of rSmTSP-2 protein and labeled with anti-CD3, and CD4 or CD8 for immunophenotyping and with anti-TNF-α, IFN-γ, IL-4 or IL-2 for detecting the production of these cytokines intracellularly by FACS. Statistical analyzes were performed by "One-way ANOVA", wherein P-value *, 0,01; **, 0,001; ***, 0,0001 are values in relation to the saline group, or between the different groups; and ns means no statistical difference.

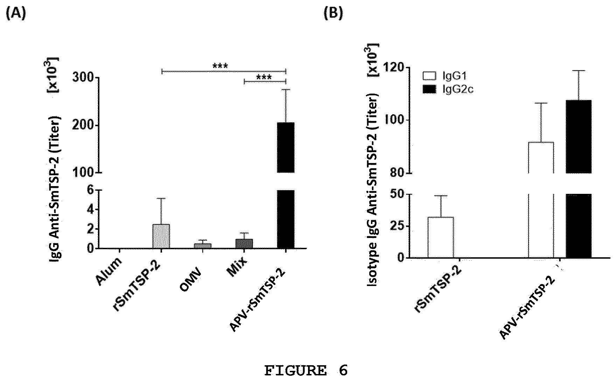

Figures 6A - B graphically depict the IgG humoral response against the rSmTSP-2 protein in mice immunized with rSmTSP-2 or APV-rSmTSP-2. Figure 6A refers to dosage of IgG anti-rSmTSP-2 antibodies in mice immunized with: Alum (Saline + Aluminum Hydroxide), rSmTSP-2 [5 µg], OMV [8 µg], OMV [8 µg] + rSmTSP-2 [2µg] (Mix) and APV-rSmTSP-2 [10 µg]). Statistical analyzes were performed by the model "Two-way ANOVA", wherein the *** P-value is < 0.001. Figure 6B refers to IgG1 and IgG2c isotypes in groups immunized with rSmTSP-2 or APV-rSmTSP-2.

Thus, the present disclosure proposes an easy and highly flexible preparation of an affinity-based antigen-presenting vesicle (APV). The APV obtained is highly specific and stable, being able to remain in the cold for months and retain its potency. Additionally, the amazing process of the present disclosure is simple enough to ensure high reproducibility, since only a few steps are required, which reduces the risk of batch-to-batch variation, with great industrial advantage.

It is also necessary to understand that the drawings are not necessarily to scale, but that they are only conceptual in nature.

## Claims

1. Process for obtaining antigen-presenting vesicles (APV) that enables the coupling of one or more antigens, **characterized by** the fact that it comprises the steps of:
a) Conjugating biotin to the outer membrane vesicle (OMV) ;
b) Obtaining rhizavidin in fusion with one or more antigen proteins or peptides; and
c) Coupling the fusion protein obtained in step "b" with the product obtained in step "a",
wherein an APV obtained comprises:
- an outer membrane vesicle (OMV) of gram-negative bacteria;
- at least one antigenic protein or peptide; and
- at least a pair of molecules with complementary affinities comprising:
(i) a biotin that binds to the OMV; and
(ii) a rhizavidin in fusion with the protein or peptide.

2. Process, according to claim 1, **characterized by** the fact that, in step "a", the conjugation reaction of the OMVs with the first affinity molecule is carried out in a suitable solution with the addition of 3 % sucrose, wherein OMVs are added in the proportion 1:1 to 1:10 (mass / mass) in relation to first affinity molecule, wherein the suitable solution used depends on the type of conjugation used, and is selected from the group consisting of buffers absent from interfering agents for conjugation, preferably a phosphate-buffered saline solution without Ca²⁺ / Mg²⁺ (PBS) or normal saline (150 mM NaCl in water).

3. Process, according to claim 1 or 2, **characterized by** the fact that, still in step "a", the mixture is maintained in the temperature of 4 to 25 °C for 4 to 18 hours, wherein the mixture subsequently goes to dialysis against the suitable solution previously used with the addition of 3 % sucrose so that the excess of the unbound first affinity molecule is eliminated.

4. Process, according to any of claims 1 to 3, **characterized by** the fact that, in step "a", optionally 0.05 M to 0.2 M of an activator molecule is used.

5. Process, according to any of claims 1 to 4, **characterized by** the fact that, in step "a", such OMVs come from bacteria selected from the group consisting of *Neisseria meningitidis* serogroup B or *Neisseria lactamica,* wherein preferably the OMVs come from *Neisseria lactamica* N.285/03.

6. Process, according to claim 4, **characterized by** the fact that, still in step "a", such activator molecule is selected from the group consisting of representative coupling agents that include organic compounds, such as thioesters, carbodiimides, succinimide esters, diisocyanatos, glutaraldehydes, diazo benzenes and hexamethylene diamines, wherein preferably such activator molecule is EDAC (1-Ethyl-3-[3-dimethylaminopropyl] carbodiimide) hydrochloride.

7. Process, according to any of claims 1 to 6, **characterized by** the fact that the biotin is linked to the OMV by a covalent bond, wherein preferably such activator molecule is used to covalently bind the biotin to the OMV.

8. Process, according to any of claims 1 to 7, **characterized by** the fact that the biotin is directly linked to the carboxyl, hydroxyl, amino, phenoxy, hemiacetal or mercapto functional groups of the OMV, without the aid of activating molecules.

9. Process, according to any of claims 1 to 8, **characterized by** the fact that, in step "b", a fusion protein is obtained comprising the antigen protein or peptide fused to the rhizavidin, wherein the rhizavidin is genetically fused to antigen proteins or peptides through the recombinant construction joining the gene of rhizavidin to the gene of the antigen or peptide of interest, wherein the construction of a chimeric sequence encoding a fusion protein is achieved by molecular biology techniques, preferably techniques selected from conventional polymerase chain reaction (PCR) or gene synthesis.

10. Process, according to any of claims 1 to 9, **characterized by** the fact that such antigen protein or peptide are selected from the group consisting of any antigen that triggers an immune response in an organism, including immunogenic peptides or proteins, toxins, toxoids, their subunits or combinations thereof; or wherein the antigen is selected from the group consisting of any antigen associated with an infectious disease or cancer or immune disease; or wherein the antigen is a component expressed by any of a variety of infectious agents, including virus, bacteria, fungus or parasite; or wherein the antigen is derived from a pathogenic organism; or wherein the antigen is a cancer or tumor antigen, such as an antigen derived from a tumor or cancer cell; or wherein the antigen is expressed by recombinant means and optionally includes an affinity tag or epitope to facilitate purification; or wherein the antigen is obtained by chemical synthesis of an oligopeptide, free or conjugated to carrier proteins.

11. Process, according to any of claims 1 to 10, **characterized by** the fact that the antigen is expressed as a fusion with a complementary affinity molecule; or wherein alternatively, the antigen is first prepared and then conjugated with a complementary affinity molecule.

12. Process, according to claim 1, **characterized by** the fact that in step "c", to carry out the coupling of the biotinylated OMVs with the fusion proteins, the OMV-biotin conjugated product is mixed with the fusion proteins obtained in step "b" in the proportion of 1:1 (mass / mass), wherein incubation takes place at a temperature ranging from 4 to 25 °C for 4 to 18 hours, and after incubation, the mixture is centrifuged at 3000 to 14000 rpm for 3 to 30 minutes to remove insoluble aggregates, wherein the supernatant is further purified.

13. Process, according to claim 12, **characterized by** the fact that the purification is carried out by ultrafiltration or chromatographic techniques.

## Patentansprüche

1. Verfahren zur Gewinnung antigenpräsentierender Vesikel (APV), die die Kopplung eines oder mehrerer Antigene ermöglichen, **dadurch gekennzeichnet, dass** es die folgenden Schritte umfasst:
a) Konjugieren von Biotin an das Außenmembranvesikel (Outer Membrane Vesicle, OMV);
b) Erhalten von Rhizavidin in Fusion mit einem oder mehreren Antigenproteinen oder -peptiden; und
c) Koppeln des in Schritt "b" erhaltenen Fusionsproteins mit dem in Schritt "a" erhaltenen Produkt,
wobei ein erhaltenes APV Folgendes umfasst:
- ein Außenmembranvesikel (OMV) von gramnegativen Bakterien;
- mindestens ein antigenes Protein oder Peptid; und
- mindestens ein Paar Moleküle mit komplementären Affinitäten, umfassend:
(i) ein Biotin, das an das OMV bindet; und
(ii) ein Rhizavidin in Fusion mit dem Protein oder Peptid.

2. Verfahren nach Anspruch 1, **dadurch gekennzeichnet, dass** in Schritt "a" die Konjugationsreaktion der OMV mit dem ersten Affinitätsmolekül in einer geeigneten Lösung unter Zusatz von 3 % Saccharose durchgeführt wird, wobei OMV im Verhältnis 1:1 bis 1:10 (Masse/Masse) in Bezug auf das erste Affinitätsmolekül zugegeben werden, wobei die verwendete geeignete Lösung von der Art der verwendeten Konjugation abhängt und aus der Gruppe bestehend aus Puffern ohne Störstoffe für die Konjugation, bevorzugt einer phosphatgepufferten Kochsalzlösung ohne Ca²⁺ / Mg²⁺ (PBS) oder normaler Kochsalzlösung (150 mM NaCl in Wasser), ausgewählt ist.

3. Verfahren nach Anspruch 1 oder 2, **dadurch gekennzeichnet, dass** noch in Schritt "a" das Gemisch 4 bis 18 Stunden bei einer Temperatur von 4 bis 25 °C gehalten wird, wobei das Gemisch anschließend eine Dialyse gegen die zuvor verwendete geeignete Lösung unter Zusatz von 3 % Saccharose durchläuft, so dass der Überschuss des ungebundenen ersten Affinitätsmoleküls entfernt wird.

4. Verfahren nach einem der Ansprüche 1 bis 3, **dadurch gekennzeichnet, dass** in Schritt "a" gegebenenfalls 0,05 M bis 0,2 M eines Aktivatormoleküls verwendet werden.

5. Verfahren nach einem der Ansprüche 1 bis 4, **dadurch gekennzeichnet, dass** in Schritt "a" solche OMV aus Bakterien stammen, die aus der Gruppe bestehend aus *Neisseria meningitidis* Serogruppe B oder *Neisseria lactamica* ausgewählt sind, wobei vorzugsweise die OMV aus *Neisseria lactamica* N.285/03 stammen.

6. Verfahren nach Anspruch 4, **dadurch gekennzeichnet, dass** noch in Schritt "a" ein solches Aktivatormolekül aus der Gruppe bestehend aus repräsentativen Kopplungsmitteln, zu denen organische Verbindungen wie Thioester, Carbodiimide, Succinimidester, Diisocyanate, Glutaraldehyde, Diazobenzole und Hexamethylendiamine gehören, wobei es sich vorzugsweise bei einem solchen Aktivatormolekül um EDAC(1-Ethyl-3-[3-dimethylaminopropyl] carbodiimid)-Hydrochlorid handelt.

7. Verfahren nach einem der Ansprüche 1 bis 6, **dadurch gekennzeichnet, dass** das Biotin über eine kovalente Bindung mit dem OMV verknüpft ist, wobei vorzugsweise ein solches Aktivatormolekül verwendet wird, um das Biotin kovalent an das OMV zu binden.

8. Verfahren nach einem der Ansprüche 1 bis 7, **dadurch gekennzeichnet, dass** das Biotin ohne die Hilfe aktivierender Moleküle direkt mit den Carboxyl-, Hydroxyl-, Amino-, Phenoxy-, Hemiacetal- oder Mercapto-Funktionsgruppen des OMV verknüpft wird.

9. Verfahren nach einem der Ansprüche 1 bis 8, **dadurch gekennzeichnet, dass** in Schritt "b" ein Fusionsprotein erhalten wird, das das an das Rhizavidin fusionierte Antigenprotein oder -peptid umfasst, wobei das Rhizavidin gentechnisch an Antigenproteine oder -peptide über die rekombinante Konstruktion, die das Gen von Rhizavidin mit dem Gen des Antigens oder Peptids von Interesse verbindet, fusioniert wird, wobei die Konstruktion einer chimären Sequenz, die ein Fusionsprotein codiert, mit molekularbiologischen Techniken, bevorzugt Techniken, die aus konventioneller Polymerase-Kettenreaktion (PCR) oder Gensynthese ausgewählt sind, erreicht wird.

10. Verfahren nach einem der Ansprüche 1 bis 9, **dadurch gekennzeichnet, dass** ein solches Antigenprotein oder - peptid aus der Gruppe bestehend aus einem beliebigen Antigen, das eine Immunantwort in einem Organismus auslöst, einschließlich immunogener Peptide oder Proteine, Toxinen, Toxoiden, deren Untereinheiten oder Kombinationen davon, ausgewählt ist; oder wobei das Antigen aus der Gruppe bestehend aus einem beliebigen Antigen, das mit einer Infektionskrankheit oder Krebs oder Immunerkrankung assoziiert ist, ausgewählt ist; oder wobei es sich bei dem Antigen um eine Komponente handelt, die durch eines aus einer Vielfalt von infektiösen Agentien, einschließlich Virus, Bakterien, Pilz oder Parasiten, exprimiert wird; oder wobei das Antigen von einem pathogenen Organismus stammt; oder wobei es sich bei dem Antigen um ein Krebs- oder Tumorantigen handelt, wie etwa ein aus einer Tumor- oder Krebszelle stammendes Antigen; oder wobei das Antigen rekombinant exprimiert wird und gegebenenfalls ein Affinitätstag oder Epitop zur Erleichterung der Aufreinigung enthält; oder wobei das Antigen durch chemische Synthese eines Oligopeptids, frei oder konjugiert an Trägerproteine, erhalten wird.

11. Verfahren nach einem der Ansprüche 1 bis 10, **dadurch gekennzeichnet, dass** das Antigen als Fusion mit einem komplementären Affinitätsmolekül exprimiert wird; oder wobei alternativ das Antigen zuerst erzeugt und dann mit einem komplementären Affinitätsmolekül konjugiert wird.

12. Verfahren nach Anspruch 1, **dadurch gekennzeichnet, dass** in Schritt "c" zur Durchführung der Kopplung der biotinylierten OMV mit den Fusionsproteinen das OMV-Biotin-konjugierte Produkt mit den in Schritt "b" erhaltenen Fusionsproteinen im Verhältnis 1:1 (Masse/Masse) gemischt wird, wobei die Inkubation 4 bis 18 Stunden bei einer Temperatur im Bereich von 4 bis 25 °C erfolgt und nach der Inkubation das Gemisch 3 bis 30 Minuten bei 3000 bis 14000 U/min zur Abtrennung unlöslicher Aggregate zentrifugiert wird, wobei der Überstand weiter gereinigt wird.

13. Verfahren nach Anspruch 12, **dadurch gekennzeichnet, dass** die Reinigung mit Ultrafiltrations- oder chromatographischen Techniken durchgeführt wird.

## Revendications

1. Procédé d'obtention de vésicules présentant un antigène (APV) qui permet le couplage d'un ou plusieurs antigènes, **caractérisé par le fait qu'**il comprend les étapes de :
a) conjugaison de biotine à la vésicule de membrane externe (OMV) ;
b) obtention de rhizavidine en fusion avec une ou plusieurs protéines ou peptides antigéniques ; et
c) couplage de la protéine de fusion obtenue à l'étape « b » avec le produit obtenu à l'étape « a »,
dans lequel une APV obtenue comprend :
- une vésicule de membrane externe (OMV) de bactéries Gram négatives ;
- au moins une protéine ou un peptide antigénique ; et
- au moins une paire de molécules avec des affinités complémentaires comprenant :
(i) une biotine qui se lie à l'OMV ; et
(ii) une rhizavidine en fusion avec la protéine ou le peptide.

2. Procédé selon la revendication 1, **caractérisé par le fait que**, dans l'étape « a », la réaction de conjugaison des OMV avec la première molécule d'affinité est effectuée dans une solution appropriée avec l'ajout de 3 % de saccharose, où les OMV sont ajoutées dans la proportion 1:1 à 1:10 (masse/masse) par rapport à la première molécule d'affinité, dans laquelle la solution appropriée utilisée dépend du type de conjugaison utilisé, et est choisie dans le groupe constitué par des tampons dépourvus d'agents interférents pour conjugaison, de préférence une solution saline tamponnée par phosphate sans Ca²⁺ / Mg²⁺ (PBS) ou une solution saline normale (NaCl 150 mM dans l'eau).

3. Procédé selon la revendication 1 ou 2, **caractérisé par le fait que**, toujours dans l'étape « a », le mélange est maintenu à une température de 4 à 25 °C pendant 4 à 18 heures, le mélange étant ensuite soumis à une dialyse contre la solution appropriée utilisée précédemment avec l'ajout de 3 % de saccharose de sorte que l'excès de la première molécule d'affinité non liée est éliminé.

4. Procédé selon l'une quelconque des revendications 1 à 3, **caractérisé par le fait que**, dans l'étape « a », on utilise éventuellement 0,05 M à 0,2 M d'une molécule activatrice.

5. Procédé selon l'une quelconque des revendications 1 à 4, **caractérisé par le fait que**, dans l'étape « a », de telles OMV proviennent de bactéries choisies dans le groupe constitué par Neisseria meningitidis sérogroupe B ou Neisseria lactamica, dans lequel de préférence les OMV proviennent de Neisseria lactamica N.285/03.

6. Procédé, selon la revendication 4, **caractérisé par le fait que**, toujours à l'étape « a », une telle molécule activatrice est choisie dans le groupe constitué par des agents de couplage représentatifs qui incluent des composés organiques, tels que des thioesters, des carbodiimides, des esters de succinimide, des diisocyanatos, des glutaraldéhydes, des diazobenzènes et des hexaméthylènediamines, ladite molécule activatrice étant de préférence le chlorhydrate d'EDAC (1-éthyl-3-[3-diméthylaminopropyl]carbodiimide).

7. Procédé selon l'une quelconque des revendications 1 à 6, **caractérisé en ce que** la biotine est liée à l'OMV par une liaison covalente, dans lequel de préférence une telle molécule activatrice est utilisée pour lier de manière covalente la biotine à l'OMV.

8. Procédé selon l'une quelconque des revendications 1 à 7, **caractérisé en ce que** la biotine est directement liée aux groupes fonctionnels carboxyle, hydroxyle, amino, phénoxy, hémiacétal ou mercapto de l'OMV, sans l'aide de molécules activatrices.

9. Procédé selon l'une quelconque des revendications 1 à 8, **caractérisé par le fait que**, dans l'étape « b », on obtient une protéine de fusion comprenant la protéine antigénique ou le peptide fusionné à la rhizavidine, dans lequel la rhizavidine est génétiquement fusionnée à des protéines ou des peptides antigéniques par la construction recombinante joignant le gène de rhizavidine au gène de l'antigène ou du peptide d'intérêt, dans lequel la construction d'une séquence chimérique codant pour une protéine de fusion est réalisée par des techniques de biologie moléculaire, de préférence, des techniques choisies parmi la réaction en chaîne par polymérase (PCR) classique ou la synthèse génique.

10. Procédé selon l'une quelconque des revendications 1 à 9, **caractérisé par le fait qu'**une telle protéine ou un tel peptide antigénique sont choisis dans le groupe constitué par tout antigène qui déclenche une réponse immunitaire dans un organisme, y compris des peptides ou protéines immunogènes, des toxines, des anatoxines, leurs sous-unités ou leurs combinaisons ; ou où l'antigène est choisi dans le groupe constitué par tout antigène associé à une maladie infectieuse ou un cancer ou une maladie immunitaire ; ou où l'antigène est un composant exprimé par l'un quelconque de différents agents infectieux, y compris virus, bactéries, champignon ou parasite ; ou où l'antigène est dérivé d'un organisme pathogène ; ou où l'antigène est un antigène de cancer ou de tumeur, tel qu'un antigène dérivé d'une cellule tumorale ou cancéreuse ; ou où l'antigène est exprimé par des moyens recombinants et comprend éventuellement une étiquette d'affinité ou un épitope pour faciliter la purification ; ou où l'antigène est obtenu par synthèse chimique d'un oligopeptide, libre ou conjugué à des protéines porteuses.

11. Procédé selon l'une quelconque des revendications 1 à 10, **caractérisé par le fait que** l'antigène est exprimé sous forme d'une fusion avec une molécule d'affinité complémentaire ; ou dans lequel, en variante, l'antigène est d'abord préparé et ensuite conjugué avec une molécule d'affinité complémentaire.

12. Procédé selon la revendication 1, **caractérisé par le fait que** dans l'étape « c », pour effectuer le couplage des OMV biotinylée avec les protéines de fusion, le produit conjugué OMV-biotine est mélangé avec les protéines de fusion obtenues dans l'étape « b » dans la proportion de 1:1 (masse/masse), où l'incubation a lieu à une température allant de 4 à 25 °C pendant 4 à 18 heures, et après incubation, le mélange est centrifugé à 3 000 à 14 000 tpm pendant 3 à 30 minutes afin d'éliminer des agrégats insolubles, le surnageant étant en outre purifié.

13. Procédé selon la revendication 12, **caractérisé par le fait que** la purification est effectuée par ultrafiltration ou des techniques chromatographiques.
